# EUROPEAN PATENT APPLICATION

(11) **EP 1 522 542 A1**
(43) Date of publication of application: **13.04.2005**
(21) Application number: 03741305.1
(22) Date of filing: 10.07.2003
(51) Int. Cl.: C07D 491/18, C07D 491/22, C07D 491/08, A61K 45/00, A61K 31/485, A61P 1/08, A61P 25/00

(54) **THERAPEUTIC OR PREVENTIVE AGENT FOR NAUSEA/VOMITING**

(30) Priority: 11.07.2002 JP 2002202657
(71) Applicant: TORAY INDUSTRIES, INC., Tokyo 103-8666 (JP)
(72) Inventor: KAWAI, Koji, Naka-gun, Kanagawa 259-0111 (JP); SAITO, Akiyoshi, Ota-ku, Tokyo 143-0014 (JP); SUZUKI, Tomohiko, Kamakura-shi, Kanagawa 248-0036 (JP); HASEBE, Ko, Kamakura-shi, Kanagawa 248-0034 (JP); SUZUKI, Tsutomu, Yokohama-shi, Kanagawa 235-0045 (JP)
(74) Representative: Hoefer & Partner
(86) International application number: PCT/JP2003/008751
(87) International publication number: WO 2004/007503

(57) **Abstract**

The present invention provides a therapeutic or prophylactic agent for preventing nausea and vomiting. The agent includes a morphinan derivative represented by general formula (I): 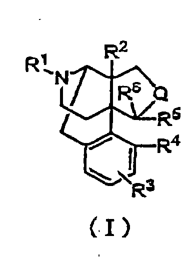 or a pharmacologically acceptable acid addition salt thereof as an active ingredient,
(where R¹ represents, for example, a cyclopropylmethyl group, R² and R³ represent, for example, a hydroxy or methoxy group, R⁴ and R⁵ together form, for example, an -O- bond, R⁶ represents, for example, a hydrogen atom, Q represents a substituted or unsubstituted group represented by, for example, the following formula: 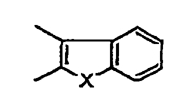 where X represents, for example, NH or NMe).

The compounds of the present invention can be widely used as medicines against vomiting caused by emetic drugs. In particular, the compounds of the present invention are useful as therapeutic or prophylactic agents for preventing nausea and vomiting caused by µ-agonists represented by morphine.

## Description

### Technical Field

The present invention relates to therapeutic or prophylactic agents for preventing nausea and vomiting. Particularly, the present invention relates to therapeutic or prophylactic agents for preventing nausea and vomiting caused by µ-opioid agonists represented by morphine.

### Background Art

Nausea and vomiting, caused by administration of emetic drugs such as opioid analgesics and anticancer drugs, are one of the most suffering side effects for patients. In particular, morphine, which is generally used as an analgesic for patients suffering from cancer pains, is known to frequently cause nausea and vomiting as adverse side effects. D₂ antagonists such as domperidone and haloperidol or anti-emetic drugs including 5-HT₃ antagonists are used for preventing nausea and vomiting due to morphine, but the anti-emetic effect of these drugs is not clear. In particular, in the early stage of morphine administration, the dosage is not sufficient to relieve the pain in many cases because of nausea and vomiting induced by morphine. This is one of the factors that make the quality of life (QOL) of patients worse. 5-HT₃ antagonists are known to exhibit a remarkable anti-emetic effect against anticancer drugs and are generally used now. However, the following problem has recently been found: The 5-HT₃ antagonists exhibit a poor effect against delayed vomiting that occurs over two or several days after the administration of the anticancer drugs. Recently, therapeutic effects of tachykinin antagonists for preventing nausea and vomiting have been reported, but they are not yet being used in practice. There is presently a demand for excellent therapeutic or prophylactic agents for preventing nausea and vomiting.

Three types of opioid receptors, i.e. µ, δ, and κ are known, and µ-receptor antagonists are reported to exhibit anti-emetic effects against vomiting due to morphine. For example, it is described in Biull. Eskp. Biol. Med. 103, 586-588 (1987) that an µ-antagonist, naloxone, shows anti-emetic effects against nausea and vomiting due to an µ-opioid agonist, morphine. However, the µ-antagonist undesirably inhibits even the analgesic effect of the µ-agonist morphine at the same time. Therefore, the clinical value of the µ-antagonist as a therapeutic agent for preventing nausea and vomiting is low.

There are practically no reports on the anti-emetic effects of δ-antagonists, but the possibility of δ-antagonists as anti-emetic agents is suggested in some literatures (for example, Eur. J. Pharmacol. 128, 143-150, (1986), US Patent Nos. 5,681,830, 5,574,159, and 5,552,404, and Lancet 1, 714-716, (1982)). On the other hand, some reports refute the anti-emetic effects of δ-antagonists (for example, Biull. Eskp. Biol. Med. 103, 586-588, (1987). This publication reports that δ-antagonist ICI-154129 cannot inhibit vomiting due to morphine.) Consequently, not all of the δ-antagonists have anti-emetic effects. There also are reports that both the µ- and δ-receptors are involved in the anti-emetic effects (for example, Biull. Eskp. Biol. Med. 103, 586-588, (1987)). Therefore, useful anti-emetic compounds may be superior to µ- and δ-antagonistic balance. However, a prediction as to whether a particular compound has such an effect is difficult. Consequently, actual synthesis and evaluation of the compound are necessary.

In general, δ-receptor antagonists weakly inhibit the analgesic effects of morphine. A compound having excellent µ- and δ-antagonistic balance is specifically suggested to be useful as an anti-emetic agent that does not inhibit the analgesic effects of morphine.

Compounds according to the present invention are already disclosed for the following uses: improvement effects against dependence and tolerance to opioids (US Patent No. 5,352,860); immunomodulation, immunosuppression, and rheumatoid therapeutic uses (PCT Publication Nos. WO 95/13071, WO 91/07966, and WO 95/17189); cocaine dependence treatment (US Patent No. 5,411,965); and antitussive uses (PCT Publication No. WO 94/14445). However, these reports do not suggest anything about the therapeutic or prophylactic effects against nausea and vomiting, as represented by the present invention.

Oxymorphone quaternary salt compounds in Japanese Unexamined Patent Application Publication No. 1-68376 and N-methylnalorphine in PCT Publication No. WO 01/13909 are reported as morphinan derivatives showing anti-emetic effects. However, the therapeutic or prophylactic effects against nausea and vomiting of indolomorphinan derivatives, quinolinomorphinan derivatives, and 7-benzylidenemorphinan derivatives according to the present invention cannot be anticipated from these reports.

### Disclosure of Invention

It is an object of the present invention to provide a therapeutic or prophylactic agent which can be widely used for preventing nausea and vomiting due to an emetic drug administration. In particular, it is an object of the present invention to provide a therapeutic or prophylactic agent for preventing nausea and vomiting due to µ-agonists represented by morphine.

With the aim of resolving the aforementioned circumstances, the inventors of the present invention have conducted intensive studies and found that a compound group consisting of particular morphinan derivatives exhibits anti-emetic activity while the side effects are mild and they barely decrease the analgesic effects of morphine.

The present invention provides that a morphinan compound represented by general formula (I): 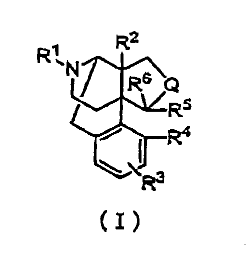 can be used as a therapeutic or prophylactic agent for preventing nausea and vomiting,
[where R¹ represents a hydrogen atom, an alkyl group having 1 to 5 carbon atoms, a cycloalkylalkyl group having 4 to 7 carbon atoms, a cycloalkenylalkyl group having 5 to 7 carbon atoms, an aryl group having 6 to 12 carbon atoms, an aralkyl group having 7 to 13 carbon atoms, an alkenyl group having 3 to 7 carbon atoms, a furanylalkyl group (where the alkyl moiety has 1 to 5 carbon atoms), or a thiophenylalkyl group (where the alkyl moiety has 1 to 5 carbon atoms); R² and R³ are mutually independent and represent a hydrogen atom, a hydroxy group, an alkoxy group having 1 to 5 carbon atoms, an alkenyloxy group having 3 to 5 carbon atoms, an aralkyloxy group having 7 to 16 carbon atoms, an arylalkenyloxy group having 7 to 16 carbon atoms, an alkanoyloxy group having 2 to 6 carbon atoms, an alkenoyloxy group having 4 to 6 carbon atoms, an arylalkanoyloxy group having 7 to 16 carbon atoms, or an alkyloxyalkoxy group having 2 to 10 carbon atoms; R⁴ and R⁵ together form an -O-, -S-, or -CH₂- bond, or are mutually independent and R⁴ represents a hydrogen atom, a hydroxy group, an alkoxy group having 1 to 5 carbon atoms, or an alkanoyloxy group having 2 to 6 carbon atoms and R⁵ represents a hydrogen atom; R⁶ represents a hydrogen atom, an alkyl group having 1 to 5 carbon atoms, an alkenyl group having 2 to 6 carbon atoms, an arylalkyl group having 7 to 16 carbon atoms, an arylalkenyl group having 7 to 16 carbon atoms, a hydroxyalkyl group having 1 to 5 carbon atoms, an alkoxyalkyl group having 2 to 12 carbon atoms, a COOH- group, or an alkoxycarbonyl group having 2 to 6 carbon atoms; and -Q- moiety represents a group as follows: 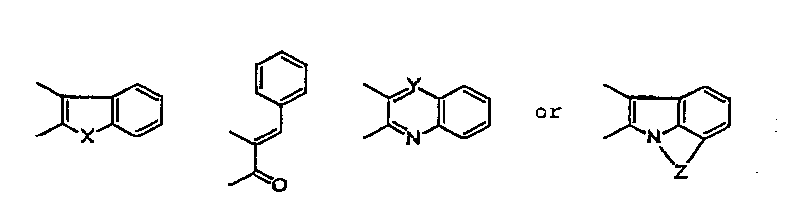 (where these structures may have one or more substituents selected from the group consisting of a fluorine atom, a chlorine atom, a bromine atom, an iodine atom, a nitro group, an alkyl group having 1 to 5 carbon atoms, a hydroxyl group, an oxo group, an alkoxy group having 1 to 5 carbon atoms, a trifluoromethyl group, a trifluoromethoxy group, a cyano group, a phenyl group, a hydroxyalkyl group having 1 to 5 carbon atoms, an isothiocyanato group, SR⁸, SOR⁸, SOOR⁸, (CH₂)ᵣOR⁸, (CH₂)ᵣCOOR⁸, SOONR⁹R¹⁰, CONR⁹R¹⁰, (CH₂)ᵣNR⁹R¹⁰, and (CH₂)ᵣN(R⁹)COR¹⁰ (where r is an integer from 0 to 5, R⁸ represents an alkyl group having 1 to 5 carbon atoms, R⁹ and R¹⁰ are mutually independent and represent a hydrogen atom, an alkyl group having 1 to 5 carbon atoms, or a cycloalkylalkyl group having 4 to 7 carbon atoms), and where X represents an oxygen atom, a sulfur atom, a CH=CH, or NR⁷ group (where R⁷ represents a hydrogen atom, an alkyl group having 1 to 5 carbon atoms, an alkenyl group having 3 to 5 carbon atoms, an arylcarbonyl group having 7 to 13 carbon atoms, an alkylsulfonyl group having 1 to 5 carbon atoms, an arylsulfonyl group having 6 to 12 carbon atoms, an aralkylsulfonyl group having 7 to 13 carbon atoms, an aralkyl group having 7 to 16 carbon atoms, an arylalkenyl group having 7 to 16 carbon atoms, an alkanoyl group having 2 to 6 carbon atoms); Y represents a nitrogen atom or a CH group; and Z represents a bridge bond having 2 to 5 carbon atoms (where one or more carbon atoms may be replaced with a nitrogen, oxygen, or sulfur atom, and an aromatic or heteroaromatic ring having 5 to 12 carbon atoms or a cycloalkyl ring having 5 to 9 carbon atoms may be fused so as to share 2 or 3 skeletal carbon atoms)].

### Brief Description of the Drawings

Fig. 1 shows the frequencies of nausea and vomiting when ferrets were subcutaneously injected with 0.1-3 mg/kg of morphine. The results are expressed as mean ± standard error of frequencies of nausea and vomiting every 30 minutes during the observation.
Fig. 2 shows the effects of NTI (5 mg/kg), which was subcutaneously administered to ferrets, against nausea and vomiting due to morphine (0.6 mg/kg) administration in the ferrets. The results are expressed as mean ± standard error of frequencies of nausea and vomiting every 30 minutes during the observation.

### Best Mode for Carrying Out the Invention

In embodiments according to the present invention, compounds represented by General Formula (I) are preferably used. In particular, preferable substituents of the compounds represented by General Formula (I) are as follows:
R¹ preferably represents a hydrogen atom, an alkyl group having 1 to 5 carbon atoms, a cycloalkylalkyl group having 4 to 7 carbon atoms, an aralkyl group having 7 to 13 carbon atoms, an alkenyl group having 3 to 7 carbon atoms, a furanylalkyl group (where the alkyl moiety has 1 to 5 carbon atoms), or a thiophenylalkyl group (where the alkyl moiety has 1 to 5 carbon atoms); more preferably represents a cycloalkylalkyl group having 4 to 7 carbon atoms or an alkenyl group having 3 to 7 carbon atoms. Specifically, a cyclopropylmethyl, cyclobutylmethyl, cyclopentylmethyl, allyl, or butenyl group is preferable. Among them, a cyclopropylmethyl or allyl group is most preferable.
R² preferably represents a hydrogen atom, a hydroxy group, an alkoxy group having 1 to 5 carbon atoms, an aralkyloxy group having 7 to 16 carbon atoms, or an alkanoyloxy group having 2 to 6 carbon atoms. Specifically, a hydrogen atom, a hydroxy, methoxy, ethoxy, propoxy, benzyloxy, phenethyloxy, acetoxy, or propanoyloxy group is preferable. Among them, a hydrogen atom, a hydroxy, methoxy, benzyloxy, or acetoxy group is most preferable.
R³ preferably represents a hydrogen atom, a hydroxy group, an alkoxy group having 1 to 5 carbon atoms, an aralkyloxy group having 7 to 16 carbon atoms, an alkanoyloxy group having 2 to 6 carbon atoms, or an arylalkanoyloxy group having 7 to 16 carbon atoms. Specifically, a hydrogen atom, a hydroxy, methoxy, ethoxy, propoxy, benzyloxy, phenethyloxy, acetoxy, propanoyloxy, or benzoyloxy group is preferable. Among them, a hydrogen atom, a hydroxy, methoxy, benzyloxy, acetoxy, or benzoyloxy group is most preferable.
Preferably, R⁴ and R⁵ together form an -O- or -S- bond, or are mutually independent and R⁴ represents a hydrogen atom, a hydroxy group, an alkoxy group having 1 to 5 carbon atoms, or an alkanoyloxy group having 2 to 6 carbon atoms and R⁵ represents a hydrogen atom. Specifically, an -O-bond formed by binding R⁴ and R⁵ is most preferable.
R⁶ preferably represents a hydrogen atom, an alkyl group having 1 to 5 carbon atoms, a COOH- group, or an alkoxycarbonyl group having 2 to 6 carbon atoms. Specifically, a hydrogen atom, a methyl, ethyl, or propyl group is preferable. Among them, a hydrogen atom or a methyl group is most preferable.

The -Q- moiety preferably represents an organic group as follows: 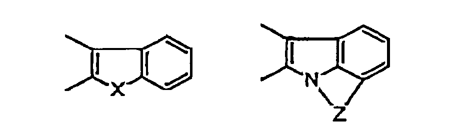
X preferably represents an oxygen atom or NR⁷ group. R⁷ preferably represents a hydrogen atom, an alkyl group having 1 to 5 carbon atoms, or an alkanoyl group having 2 to 6 carbon atoms. Specifically, a hydrogen atom, a methyl, ethyl, propyl, butyl, acetyl, or propanoyl group is preferable. Among them, a hydrogen atom, a methyl or acetyl group is most preferable.
Z preferably represents a bridge bond having 2 to 5 carbon atoms (where one or more carbon atoms may be replaced with a nitrogen, oxygen, or sulfur atom). Specifically, a -(CH₂)₂-, -(CH₂)₃-, - (CH₂)₄-, -(CH₂)₅-, -(CH₂)₂-O-, -(CH₂)₂-S-, or -(CH₂)₂-NR⁷- group is preferable (where R⁷ preferably or -(CH₂)₂-NR⁷- group is preferable (where R⁷ preferably represents a hydrogen atom, an alkyl group having 1 to 5 carbon atoms, or an alkanoyl group having 2 to 6 carbon atoms, specifically, a hydrogen atom, a methyl, ethyl, propyl, butyl, acetyl, or propanoyl group is preferable, and among them, a hydrogen atom, a methyl or acetyl group is most preferable).

Preferably, the substituent of the organic group is a fused benzene, fused pyridine, fused cyclohexane, or fused cyclopentane ring, a fluorine, chlorine, bromine, or iodine atom, a nitro group, an alkyl group having 1 to 5 carbon atoms, a hydroxyl or oxo group, an alkoxy group having 1 to 5 carbon atoms, a trifluoromethyl, trifluoromethoxy, cyano, or phenyl group, a hydroxyalkyl group having 1 to 5 carbon atoms, an isothiocyanato group, an SR⁸, SOR⁸, SOOR⁸, (CH₂)ᵣOR⁸, (CH₂)ᵣCOOR⁸, SOONR⁹R¹⁰, CONR⁹R¹⁰, (CH₂)ᵣ NR⁹R¹⁰, or (CH₂)ᵣN(R⁹)COR¹⁰ group (where r is an integer from 0 to 5, R⁸ represents an alkyl group having 1 to 5 carbon atoms, R⁹ and R¹⁰ are mutually independent and represent a hydrogen atom, an alkyl group having 1 to 5 carbon atoms, or a cycloalkylalkyl group having 4 to 7 carbon atoms). Specifically, a fused benzene, fused cyclohexane, or fused cyclopentane ring, a fluorine, chlorine, bromine, or iodine atom, a nitro, methyl, ethyl, isopropyl, hydroxy, oxo, methoxy, ethoxy, trifluoromethyl, trifluoromethoxy, cyano, phenyl, hydroxymethyl, hydroxyethyl, isothiocyanato, methylthio, ethylthio, methylsulfinyl, ethylsulfinyl, methylsulfonyl, ethylsulfonyl, methoxymethyl, ethoxymethyl, methoxycarbonyl, ethoxycarbonyl, methoxycarbonylmethyl, ethoxycarbonylmethyl, sulfamoyl, dimethylsulfamoyl, dimethylcarbamoyl, dimethylamino, dimethylaminomethyl, or amino group is preferable. An unsubstituted organic group is also preferably included in the present invention. However, the present invention is not limited to these substituents.

Examples of pharmacologically preferable acid addition salts include inorganic salts such as hydrochloride, sulfate, nitrate, hydrobromate, hydroiodate, and phosphate; organic carboxylate salts such as acetate, lactate, citrate, oxalate, glutarate, malate, tartrate, fumarate, mandelate, maleate, benzoate, and phthalate; and organic sulfonate salts such as methanesulfonate, ethanesulfonate, benzenesulfonate, p-toluenesulfonate, and camphorsulfonate. Among them, hydrochloride, hydrobromate, phosphate, tartrate, methanesulfonate are most preferable, but the present invention is not limited to these salts.

Compounds represented by General Formula (I) of the present invention can be prepared by processes described in, for example, PCT Publication Nos. WO 94/14445, WO 97/11948, NO 89/00995, and WO 95/31463, and Heterocycles, 45, 2109, (1997).

The anti-emetic effects of the compounds represented by General Formula (I) can be evaluated by a method described in a publication (Eur. J. Pharmacol. 374 (1999), 77-84), but the evaluation procedure is not limited to this method.

The compounds represented by General Formula (I) according to the present invention are useful for controlling vomiting due to radiotherapy for cancer, a toxic agent, a toxin, metabolic disorder (for example, gastritis), hyperemesis, rotatory vertigo, kinetosis (for example, carsickness), postoperative sequelae, gastrointestinal dysfunction, gastrointestinal hypokinesia, visceral pain (for example, myocardial infarction and peritonitis), migraine, an increase in intra-cranial pressure, and a decrease in intra-cranial pressure such as altitude sickness. The term "vomiting" includes nausea, emesis, and vomiting. Vomiting includes acute vomiting, delayed vomiting, and premonitory vomiting.

Specifically, the compounds represented by General Formula (I) according to the present invention are significantly useful for controlling vomiting due to the following emetic drugs:
(1) Anticancer drugs (antineoplastic agents): For example, cyclophosphamide, carmustine, lomustine, chlorambucil, busulfan, melfalan, mecloretamine, vinca alkaloids (e.g., etoposide, vinblastine, vincristine, etc.), ergot alkaloids (e.g., ergot alkaloid, bromocriptine, etc.), fumagillin derivatives (e.g., (3R,4S,5S,6R)-5-methoxy-4-[(2R,3R)-2-methyl-3-(3-methyl-2-butenyl)oxiranyl]-1-oxaspiro[2,5]oct-6-yl(chloroacetyl)carbamate), methotrexate, emethine, mustine, cysplatine, dacarbazine, procarbazine, dactinomycin, doxorubicin, mytomycin C, bleomycin, asparaginase, daunorubicin, floxuridine, cytarabine, fluorouracil, mercaptopurine, mitotane, procarbazine, streptozocin, tamoxifen, thioguanine, and busulfan.
(2) Antibiotics: For example, erythromycin and its derivatives [e.g., erythromycin such as erythromycin A, B, C, and D and their derivatives (e.g., N-demethyl-N-isopropyl-8,9-anhydroerythromycin A-6,9-hemiacetal), clarithromycin], aminoglycoside (e.g., streptomycin, neomycin, gentamicin), actinomycin, adriamycin, and cycloheximide.
(3) Morphine, its derivatives, and its salts [e.g., opioid analgesics such as morphine and its salts, therapeutic drugs for male erectile dysfunction (impotence) such as apomorphine and its salts, and therapeutic drugs for Parkinson's disease (dopamine D₂-receptor agonists)].
(4) Others: antidiarrheals such as opioid-receptor agonists (e.g., loperamide), antineoplastic agents (e.g., hydroxyurea), antiasthmatics such as phosphodiesterase IV inhibitors (e.g., rolipram), histamine, pilocarpine, protoveratrine, levodopa, theophylline, hydroxycarbamide, thiotepa, carboplatine, epirubicin, etc.

The emetic drugs include a combination of the above-mentioned agents (preferably, a combination of two or three agents). The compounds represented by General Formula (I) according to the present invention are effective for preventing vomiting caused by µ-opioid agonists among the above-mentioned agents, and are preferably used for preventing vomiting due to opioid analgesics such as morphine, its derivatives, and its salts, or due to antidiarrheals such as opioid-receptor agonists (e.g., loperamide), specifically due to opioid analgesics such as morphine, its derivatives, and its salts.

The compounds represented by General Formula (I) are less toxic and safer, and are useful as anti-emetics for mammals (for example, hamster, feline, ferret, canine, bovine, ovine, simian, and human).

The therapeutic or prophylactic agents for preventing nausea and vomiting according to the present invention are mixed with pharmacologically acceptable carriers and are administered orally or parenterally as, for example, solid pharmaceutical preparations such as powders, granules, tablets, and capsules; liquid pharmaceutical preparations such as syrups, emulsions, and injections (hypodermic injections, intravenous injections, intramuscular injections, and drip infusions); sustained-release preparations such as sublingual tablets, buccals, troches, and microcapsules; intraoral fast-dissolving preparations, or suppositories. These dosage forms are prepared by known pharmaceutical preparation technologies.

Examples of the pharmacologically acceptable carriers include various organic and inorganic carrier substances which are generally used as pharmaceutical materials. These carriers are used in solid pharmaceutical preparations as excipients, lubricants, binders, or disintegrators; in liquid pharmaceutical preparations as solvents, solubilizers, suspending agents, isotonizing agents, buffers, or soothing agents. If necessary, pharmaceutical additives such as preservatives, antioxidants, coloring agents, and edulcorants may be used.

Preferable examples of the excipients include lactose, sucrose, D-mannitol, starch, crystalline cellulose, light anhydrous silicic acid, calcium carbonate, and calcium phosphate.

Preferable examples of the lubricants include stearic acid, magnesium stearate, calcium stearate, talc, and colloidal silica. Preferable examples of the binders include crystalline cellulose, sucrose, D-mannitol, dextrine, hydroxypropylcellulose, hydroxypropylmethylcellulose, polyvinyl pyrrolidone, gum Arabic, and gelatin.

Preferable examples of the disintegrators include starch, carboxymethylcellulose, calcium carboxymethylcellulose, sodium croscarmellose, and sodium carboxymethyl starch. Preferable examples of the solvents include an injection solvent, saline, alcohol, propylene glycol, macrogol, sesame oil, and maize oil.

Preferable examples of the solubilizers include polyethylene glycol, propylene glycol, D-mannitol, benzyl benzoate, ethanol, tris-aminomethane, cholesterol, triethanolamine, sodium carbonate, and sodium citrate.

Preferable examples of the suspending agents include surfactants such as stearyltriethanolamine, sodium lauryl sulfate, laurylaminopropionic acid, lecithin, benzalkonium chloride, benzethonium chloride, and glyceryl monostearate; and hydrophilic polymers such as polyvinyl alcohol, polyvinylpyrrolidone, sodium carboxymethyl cellulose, methylcellulose, hydroxymethyl cellulose, hydroxyethyl cellulose, and hydroxypropyl cellulose.

Preferable examples of the isotonizing agents include sodium chloride, glycerin, and D-mannitol.

Preferable examples of the buffers include buffer solutions of phosphate salts, acetate salts, carbonate salts, and citrate salts. Preferable examples of the soothing agents include benzyl alcohol.

Preferable examples of the preservatives include paraoxybenzoic acid esters, chlorobutanol, benzyl alcohol, phenethyl alcohol, dehydroacetic acid, and sorbic acid.

Preferable examples of the antioxidants include sulfites and ascorbic acid.

The compounds according to the present invention can be used with the above-mentioned emetic agents (1) in the forms of pharmaceutical preparations (pharmaceutical compositions) containing a compound represented by General Formula (I) and an emetic agent, or (2) by preparing the compound represented by General Formula (I) and the emetic drug and by separately or simultaneously prescribing the compound represented by General Formula (I) and the emetic drug. Doses of the compounds of the present invention are appropriately determined in accordance with symptom severity, subject age, weight, and route of administration. An effective daily dose for an adult ranges from 0.1 µg to 100 g, preferably from 1 µg to 1 g, and more preferably from 10 µg to 100 mg, and is administered in one dose or split into two or more separate doses in a day.

When the compounds of the present invention are used as therapeutic or prophylactic agents for preventing nausea and vomiting, the following agents may be simultaneously used with the compounds of the present invention for reducing vomiting, preventing vomiting, and increasing anti-emetic efficacy: autonomic blocking agents; anti-dopamine agents; serotonin antagonists [for example, ondansetron (Zofran) and its sustained-release preparation (Zofran Zydis), granisetron (Kytril), azasetron (Serotone), ramosetron (Nasea), metoclopramide (Primperan) and its sustained-release preparation (Pramidin), tropisetron (Novoban), mosapride (Gasmotin), dolasetron (Anemet), palonosetron (RS-42358-197), itasetron (U-98079A), indisetron (N-3389), KAE-393, R-zacopride (SL-920241), lerisetron (F-0930-RS), E-3620, and Ro-93777]; histamine antagonists; parasympatholytic agents; anti-emetic agents (for example, metopimazine, trimethobenzamide, benzquinamine hydrochloride, and diphenidol hydrochloride); glucosteroids [for example, cortisone acetate (Cortone), hydrocortisone (Cortril, Hydrocortone) and its sodium phosphate salt (hydrocortisone soluble), dexamethazone (Corson, Decaderon, Decaderon S), its acetate salt (Decadron A injection aqueous suspension, etc.), its sodium phosphate salt (Decadron, Decadron injection solution, Decadron S), and its sodium sulfate salt (Dexa-Scheroson injection solution, Dexa-Scheroson injection solution B), betamethasone (Rinderon, Betonelan), its sodium phosphate salt (Rinderon), and its acetate salt (Rinderon suspension), prednisolone (Predonine, prednisolone tablets, etc.), its acetate salt (Predonine soluble), its sodium phosphate salt (Dojilon injection), its butyl acetate salt (Codelcortone), and its sodium succinate salt (Predonine soluble), methylpredonine (Medrol), its acetate salt (Depo-Medrol), and its sodium succinate salt (Sol-Medrol), triamcinolone acetate (Kenacort), and other adrenocortical steroids such as steroids having glucocorticoid activities, and their salts]; psycholeptics such as chlorpromazine; and tranquilizers. In particular, serotonin antagonists such as ondansetron; and glucosteroids such as dexamethasone, its acetate salt, its sodium phosphate salt, and its sodium sulfate salt are preferably used together.

The compounds of the present invention can be used as pharmaceutical preparations (pharmaceutical compositions) containing a compound represented by General Formula (I) and a concomitant drug such as the above-mentioned serotonin antagonists.

The compounds of the present invention can be used in the forms of pharmaceutical preparations (pharmaceutical compositions) (1) containing a compound represented by General Formula (I), an emetic agent, and a serotonin antagonist, (2) containing a compound represented by General Formula (I), an emetic drug, and glucosteroid, or (3) containing a compound represented by General Formula (I), an emetic drug, a serotonin antagonist, and glucosteroid.

Medicines according to the present invention may be simultaneously administered with the emetic drugs. An emetic drug is administered in advance and then a compound represented by General Formula (I) may be administered before or after the onset of vomiting, or the compound represented by General Formula (I) is administered in advance and then the emetic drug may be administered. When there is a time interval between the administrations of the compound and the emetic drug, the time interval is determined in accordance with the active substance, the dosage form, and the administration method. For example, when an emetic drug is administered in advance, a compound represented by General Formula (I) is administered between one minute and three days after the administration of the emetic drug, preferably between ten minutes and one day, more preferably between fifteen minutes and one hour after the administration of the emetic drug. When a compound represented by General Formula (I) is administered in advance, an emetic drug is administered between one minute and one day after the administration of the compound represented by General Formula (I), preferably between ten minutes and six hours, more preferably between fifteen minutes and one hour after the administration of the compound represented by General Formula (I). When a medicine according to the present invention is administered with a serotonin antagonist or glucosteroid, the serotonin antagonist and the glucosteroid may be simultaneously administered with the compound represented by General Formula (I) or may be administered at different times. When these drugs are administered at different times, for example, when an emetic drug is administered in advance, the drugs are administered between one minute and three days after the administration of the emetic drug, preferably between ten minutes and one day, more preferably between fifteen minutes and one hour after the administration of the emetic drug.

### EXAMPLES

The present invention will now be explained in detail with reference to COMPARATIVE EXAMPLES and EXAMPLES hereinafter.

### COMPARATIVE EXAMPLE 1

Preparation of NTI (Naltrindole, 1):
Naltrexone hydrochloride (150 g) was suspended in ethanol (2.5 L), and then phenylhydrazine (45.1 g) and
methanesulfonate (382 g) were added. The mixture was heated under reflux for 2 hours. The mixture was cooled to room temperature and was left over night to precipitate a solid substance. The solid substance was collected by filtration and was then washed with a small amount of ethanol (yield of crude product: 156.87 g, 77%). The crude product was dissolved in methanol (4.3 L) and the solution was concentrated to about 3 L to crystallize at room temperature (104.45 g, 52%). The mother liquor was recrystallized to recover the target product at a total yield of 73% (second crystallization: 33.92 g, 17%; third crystallization: 9.10 g, 4.5%). λmax 225, 282, EIMS m/z 414 (M+).

### COMPARATIVE EXAMPLE 2

Preparation of methanesulfonate salt of 17-(cyclopropylmethyl)-6,7-didehydro-4,5α-epoxy-3,14β-dihydroxy-7'-methylsulfinyl-6,7-2',3'-indolomorphinan highly polar compound (Compound 2):

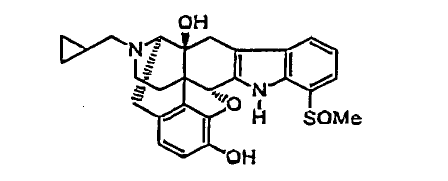

The compound was prepared by a method described in Example 16 of PCT Publication No. WO 94/07896.

### COMPARATIVE EXAMPLE 3

Preparation of methanesulfonate salt of 17-(cyclopropylmethyl)-6,7-didehydro-4,5α-epoxy-3,14β-dihydroxy-1',7'-trimethylene-5'-fluoro-6,7-2',3'-indolomorphinan (Compound 3):

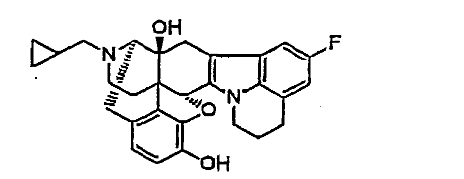

The compound was prepared by a method described in Example 10 of PCT Publication No. WO 97/11948.

### COMPARATIVE EXAMPLE 4

Preparation of methanesulfonate salt of 17-(cyclopropylmethyl)-6,7-didehydro-4,5α-epoxy-3,14β-dihydroxy-6',7'-cyclohexeno-1'-methyl-6,7-2',3'-indolomorphinan (Compound 4):

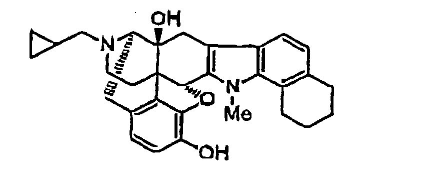

The compound was prepared by a method described in Heterocycles, 45, 2109, (1997).

### COMPARATIVE EXAMPLE 5

Preparation of methanesulfonate salt of 17-(cyclopropylmethyl)-6,7-didehydro-4,5α-epoxy-3,14β-dihydroxy-6,7-2',3'-quinolinomorphinan (Compound 5):

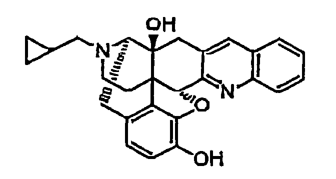

The compound was prepared by a method described in Comparative Example 1 of PCT Publication No. WO 98/43977.

### COMPARATIVE EXAMPLE 6

Preparation of methanesulfonate salt of 17-(cyclopropylmethyl)-6,7-didehydro-4,5α-epoxy-3,14β-dihydroxy-6,7-2',3'-(4'-methylquinolino)morphinan (Compound 6) :

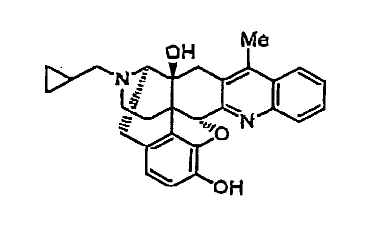

The compound was prepared by a method described in Example 19 of PCT Publication No. WO 98/43977.

### COMPARATIVE EXAMPLE 7

Preparation of 7-benzylidene-17-(cyclopropylmethyl)-4,5α-epoxy-3,14β-dihydroxy-6-oxomorphinan (Compound 7):

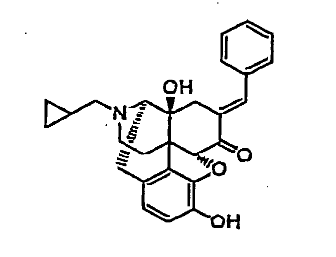

The compound was prepared by a method described in Example 1 of PCT Publication No. WO 93/21188.

### EXAMPLE 1

The inhibitory effects of NTI on morphine-induced nausea and vomiting:

Male ferrets were used for the experiment. Frequencies of nausea and vomiting were visually observed from immediately after the administration of morphine to one hour after the administration.

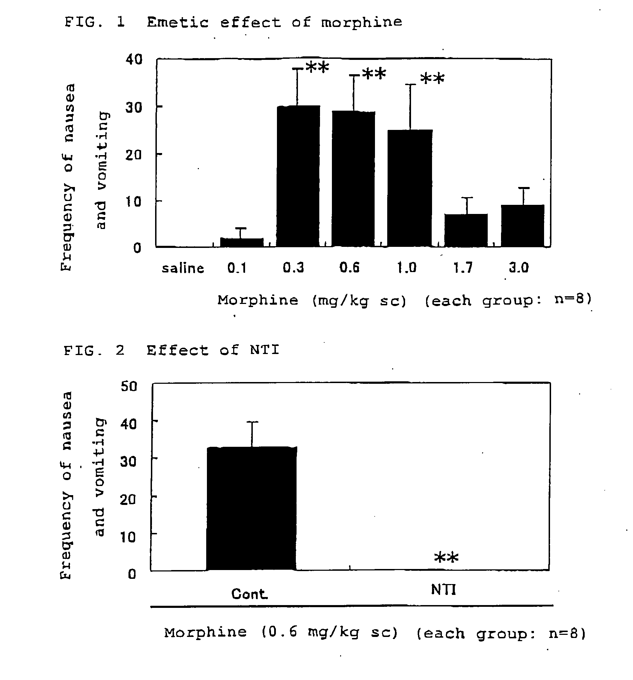

Morphine (0.1-3 mg/kg) was subcutaneously administered to male ferrets. Nausea and vomiting were remarkably induced at lower doses. The inhibitory effects of NTI on nausea and vomiting due to morphine were then studied. NTI (5 mg/kg) was subcutaneously administered 30 minutes before the administration of morphine (0.6 mg/kg). The inhibitory effects of NTI on morphine-induced nausea and vomiting are shown in Figs. 1 and 2.

### EXAMPLE 2

The inhibitory effects of the test compounds on morphine-induced nausea and vomiting:

Male beagles were used for the experiment. Frequencies of nausea and vomiting were visually observed for ten minutes after the administration of morphine.

Morphine (0.5 mg/kg) was intravenously administered into the foreleg of male beagles. Nausea and vomiting were remarkably induced within 5 minutes after the administration. The test compounds were intravenously administered into the foreleg 15 minutes before the administration of morphine. Doses at which the test compounds inhibited nausea and vomiting due to morphine are shown in Table 1.

**Table 1**

| Anti-emetic effect of test compounds | |
|---|---|
| Test compounds | dose (mg/kg) |
| Compound 2 (COMPARATIVE EXAMPLE 2) | 1.0 |
| Compound 3 (COMPARATIVE EXAMPLE 3) | 1.0 |
| Compound 4 (COMPARATIVE EXAMPLE 4) | 3.0 |
| Compound 5 (COMPARATIVE EXAMPLE 5) | 1.0 |
| Compound 6 (COMPARATIVE EXAMPLE 6) | 3.0 |
| Compound 7 (COMPARATIVE EXAMPLE 7) | 1.0 |

The results show that the test compounds have significant effects in improving the prevention of nausea and vomiting due to morphine administration.

## Claims

1. A therapeutic or prophylactic agent for preventing nausea and vomiting, the agent comprising a morphinan derivative represented by general formula (I): 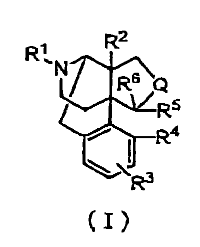 or a pharmacologically acceptable acid addition salt thereof as an active ingredient,
[where R¹ represents a hydrogen atom, an alkyl group having 1 to 5 carbon atoms, a cycloalkylalkyl group having 4 to 7 carbon atoms, a cycloalkenylalkyl group having 5 to 7 carbon atoms, an aryl group having 6 to 12 carbon atoms, an aralkyl group having 7 to 13 carbon atoms, an alkenyl group having 3 to 7 carbon atoms, a furanylalkyl group (where the alkyl moiety has 1 to 5 carbon atoms), or a thiophenylalkyl group (where the alkyl moiety has 1 to 5 carbon atoms); R² and R³ are mutually independent and represent a hydrogen atom, a hydroxy group, an alkoxy group having 1 to 5 carbon atoms, an alkenyloxy group having 3 to 5 carbon atoms, an aralkyloxy group having 7 to 16 carbon atoms, an arylalkenyloxy group having 7 to 16 carbon atoms, an alkanoyloxy group having 2 to 6 carbon atoms, an alkenoyloxy group having 4 to 6 carbon atoms, an arylalkanoyloxy group having 7 to 16 carbon atoms, or an alkyloxyalkoxy group having 2 to 10 carbon atoms; R⁴ and R⁵ together form an -O-, -S-, or -CH₂- bond, or are mutually independent and R⁴ represents a hydrogen atom, a hydroxy group, an alkoxy group having 1 to 5 carbon atoms, or an alkanoyloxy group having 2 to 6 carbon atoms and R⁵ represents a hydrogen atom; R⁶ represents a hydrogen atom, an alkyl group having 1 to 5 carbon atoms, an alkenyl group having 2 to 6 carbon atoms, an arylalkyl group having 7 to 16 carbon atoms, an arylalkenyl group having 7 to 16 carbon atoms, a hydroxyalkyl group having 1 to 5 carbon atoms, an alkoxyalkyl group having 2 to 12 carbon atoms, a COOH- group, or an alkoxycarbonyl group having 2 to 6 carbon atoms; and -Q- moiety represents a group as follows: 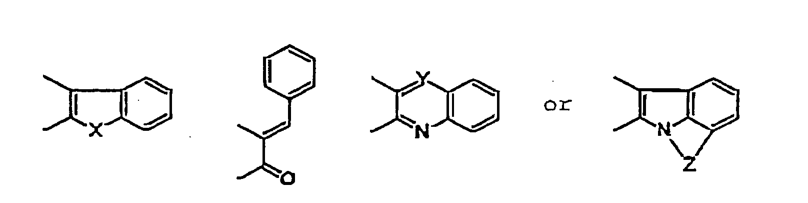 (where these structures may have one or more substituents selected from the group consisting of a fluorine atom, a chlorine atom, a bromine atom, an iodine atom, a nitro group, an alkyl group having 1 to 5 carbon atoms, a hydroxyl group, an oxo group, an alkoxy group having 1 to 5 carbon atoms, a trifluoromethyl group, a trifluoromethoxy group, a cyano group, a phenyl group, a hydroxyalkyl group having 1 to 5 carbon atoms, an isothiocyanato group, SR⁸, SOR⁸, SOOR⁸, (CH₂)ᵣOR⁸, (CH₂)ᵣCOOR⁸, SOONR⁹R¹⁰, CONR⁹R¹⁰, (CH₂)ᵣNR⁹R¹⁰, and (CH₂)ᵣN(R⁹)COR¹⁰ (where r is an integer from 0 to 5, R⁸ represents an alkyl group having 1 to 5 carbon atoms, R⁹ and R¹⁰ are mutually independent and represent a hydrogen atom, an alkyl group having 1 to 5 carbon atoms, or a cycloalkylalkyl group having 4 to 7 carbon atoms), and where X represents an oxygen atom, sulfur atom, a CH=CH, or NR⁷ group (where R⁷ represents a hydrogen atom, an alkyl group having 1 to 5 carbon atoms, an alkenyl group having 3 to 5 carbon atoms, an arylcarbonyl group having 7 to 13 carbon atoms, an alkylsulfonyl group having 1 to 5 carbon atoms, an arylsulfonyl group having 6 to 12 carbon atoms, an aralkylsulfonyl group having 7 to 13 carbon atoms, an aralkyl group having 7 to 16 carbon atoms, an arylalkenyl group having 7 to 16 carbon atoms, an alkanoyl group having 2 to 6 carbon atoms); Y represents a nitrogen atom or a CH group; and Z represents a bridge bond having 2 to 5 carbon atoms (where one or more carbon atoms may be replaced with a nitrogen, oxygen, or sulfur atom, and an aromatic or heteroaromatic ring having 5 to 12 carbon atoms or a cycloalkyl ring having 5 to 9 carbon atoms may be fused so as to share 2 or 3 skeletal carbon atoms)].

2. The therapeutic or prophylactic agent for preventing nausea and vomiting according to claim 1, wherein the -Q-moiety in general formula (I) represents a group: 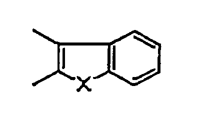 (where X is as defined above and the group may have the substituents above).

3. The therapeutic or prophylactic agent for preventing nausea and vomiting according to claim 1, wherein the -Q-moiety in general formula (I) represents a group: 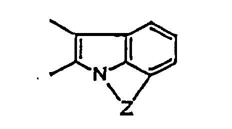 (where Z is as defined above and the group may have the substituents above).

4. The therapeutic or prophylactic agent for preventing nausea and vomiting according to claim 4, wherein R⁴ and R⁵ in general formula (I) together form an -O- bond.

5. The therapeutic or prophylactic agent for preventing nausea and vomiting according to any one of claims 1 to 4, wherein the agent prevents nausea and vomiting caused by a µ-opioid agonist compound.

6. The therapeutic or prophylactic agent for preventing nausea and vomiting according to claim 5, wherein the µ-opioid agonist compound is morphine.
